# EUROPEAN PATENT SPECIFICATION

(11) **EP 3 861 978 B1**
(45) Date of publication and mention of the grant of the patent: **17.07.2024**
(21) Application number: 19869265.9
(22) Date of filing: 04.06.2019
(51) Int. Cl.: A61K 8/73, A61K 8/02, A61K 8/81, A61K 8/87, A61K 8/06, A61Q 1/14

(54) **MAKEUP BASE COSMETIC COMPOSITION FOR SKIN**
KOSMETISCHE ZUSAMMENSETZUNG ZUM SCHMINKEN DER HAUT
COMPOSITION COSMÉTIQUE DE BASE DE MAQUILLAGE POUR LA PEAU

(30) Priority: 04.10.2018 KR 20180118110
(43) Date of publication of application: 11.08.2021
(73) Proprietor: LG Household & Health Care Ltd., Seoul 03184 (KR)
(72) Inventor: JUN, Seung Hyun, Seoul 07795 (KR); KIM, Seo Yeon, Seoul 07795 (KR)
(74) Representative: Goddar, Heinz J.
(86) International application number: PCT/KR2019/006745
(87) International publication number: WO 2020/071613

(56) References cited:
- JP-A- 2017 048 293
- KR-A- 20160 146 259
- KR-A- 20170 060 744
- KR-A- 20180 016 925
- KR-B1- 101 791 840
- DATABASE WPI Week 201764, Derwent World Patents Index; AN 2017-621074, XP002806677

## Description

### [Background Art]

Most makeup products contain a large amount of oily ingredients and colorants such as pigments, which are hydrophobic substances, so that when cleansing, it is necessary to use cleansing products containing oily ingredients, such as cleansing oils and creams, as well as cleansing foam. However, such a cleansing process can irritate the skin, so that consumers want a quicker and simpler cleansing method.

In order to solve the problems described above, a film-forming polymer or a temperature-sensitive polymer has been used conventionally. The film-forming polymer forms a film on the skin and increases cleansing power through structural changes that occur when it contacts water. The temperature-sensitive polymers form a hydrophobic film below a specific temperature, and when a certain temperature is reached, the structure is changed and the film is detached from the skin, thus making it possible to cleanse makeup. However, in the case of cleansing makeup using the polymers as described above, it is difficult to completely remove even makeup that is clogged in pores or wrinkles, and the cleansing effect may be degraded because the polymers fall off in small units.

Accordingly, there has a demand for research and development on cosmetic compositions that facilitate makeup cleansing.

In general, cellulose generally used as a raw material for cosmetics includes natural cellulose, cellulose derivatives, biocellulose, and the like.

Natural cellulose is in the form of a powder with a size of several tens of micrometers, is insoluble in water, and is mainly used for scrubs, peeling gels, and the like.

Cellulose derivatives can be obtained by substituting cellulose with a hydroxy alcohol group, and since cellulose itself is not soluble in water, there are hydroxyethyl cellulose, carboxymethyl cellulose, and the like modified to be soluble in water. This water-soluble cellulose is in the form of a polymer and is mainly used as a thickener.

Biocellulose is a cellulose microfibril synthesized from bacterial cellulose and has a small fiber thickness, high crystallinity, and high physical strength compared to plant-derived cellulose, but is mainly used for a mask pack sheet because it is synthesized in the form of a gel or sheet. Particularly, biocellulose has a disadvantage of being difficult to apply to cosmetic formulations, and thus, to apply biocellulose to cosmetics, a process of dispersing biocellulose in an aqueous solution while maintaining the microfibril form is essentially required.

For this dispersion process, in Patent Document 1 (Korean Patent Publication No. 10-2017-0103698 (September 13, 2017)) as the previous study, a raw material derived from the biocellulose fiber sheet was chemically treated with sodium hypochlorite, bromine chloride, and 2,2,6,6-tetramethyl-1-piperidine-N-oxy radical (TEMPO) as a catalyst to make water dispersion possible. Specifically, Patent Document 1 discloses the effect of increased skin adhesion, moisture-holding capacity, moisture retention power, and elasticity of the microfibril dispersion in which biocellulose is dispersed in water, but it does not disclose the effect related to cleansing makeup.

WO 2017/150950 A1 discloses a cosmetic composition comprising biocellulose microfibers in the form of an aqueous network dispersion wherein the fibers have an average diameter of 30-60 nm and a maximum diameter of 60-100 nm.

### [Disclosure]

### [Technical Problem]

As a result of researching to develop cosmetics that are easy to cleanse makeup, the present inventors found that when biocellulose microfibrils are applied before applying color cosmetics, makeup cleansing power is improved due to desorption of a film formed by application of the microfibrils, and thus completed the present invention.

Accordingly, an object of the present invention is to provide a method of improving the removal efficiency of a color cosmetic that facilitates cleansing of makeup.

### [Technical Solution]

One aspect of the present invention provides a method of improving the removal efficiency of a color cosmetic, comprising applying a makeup base cosmetic composition for skin containing biocellulose microfibrils to the skin before applying the color cosmetic, wherein the biocellulose microfibrils have a number-average diameter of 30 to 60 nm and a maximum diameter of 60 to 100 nm, and wherein the cosmetic composition further comprises a polyvinyl-alcohol-based film-forming polymer.

In the present invention, the "makeup base for skin" refers to a makeup base applied first to form a physical film before applying a color cosmetic to the skin. Provided is a cosmetic composition that facilitates cleansing of the color cosmetic applied on the film while the physical film formed from the biocellulose microfibrils is detached.

Hereinafter, the configuration of the present invention will be described in detail.

The makeup base cosmetic composition for skin includes biocellulose microfibrils.

The biocellulose microfibrils may be applied in the form of a biocellulose microfibril network dispersion, and the dispersion may be applied to the cosmetic composition as a biocellulose microfibril dispersion in which the dispersion is dispersed in an aqueous solution.

In the present invention, the biocellulose microfibril network dispersion is characterized in that a hydroxyl group of biocellulose is substituted with a carboxyl group, and a three-dimensional network is formed while the biocellulose microfibrils are being dispersed in an aqueous phase. For example, among all hydroxy groups contained in biocellulose, 0.8 mmol or more, and preferably 1.0 mmol or more may be substituted with a carboxyl group. The substitution of the hydroxy group of biocellulose with a carboxyl group may be performed by various methods known in the art, and for example, the substitution can be carried out through a process of adding and stirring an oxidizing agent and biocellulose together to distilled water in which an N-oxyl compound is dissolved.

The dispersion uses biocellulose as a cellulose raw material. The 'biocellulose' is a bacterial cellulose in which cellulose microfibrils are directly synthesized through bacterial culture, and the biocellulose is distinguished from powdered cellulose produced by pulverizing various types of wood-derived kraft paper or sulfurous acid pulp, powdered cellulose obtained by pulverizing the kraft paper or sulfurous acid pulp with a homogenizer or mill, microcrystalline cellulose powder obtained by purifying the kraft paper or sulfurous acid pulp through chemical treatment such as acid hydrolysis, or cellulose derived from plants such as kenaf, hemp, rice, Bacchus, and bamboo. Bacteria that may be used for the synthesis of cellulose include, for example, the *Acetobacter* genus, the *Rhizibium* genus, and the *Agrobacterium* genus, and several kinds of bacteria may be mixed and cultured for production efficiency. When the bacteria are cultured, bacterial cellulose is formed at the interface of the culture solution. Methods of culturing the cellulose include static cultivation and agitated cultivation. In the static cultivation method, bacteria are first inoculated into a medium and then maintained in a flask on a shelf for about ten days for cultivation. The agitated cultivation method is a culturing method in which bacteria are cultured in a liquid medium in a shaking incubator while continuously stirring at a constant rate. Biocellulose has a three-dimensional network, high crystallinity (84 to 89%), and sufficient porosity. The length of the biocellulose is several to tens of micrometers, and the biocellulose has a constant diameter, that is, a diameter with even length distribution.

In one embodiment, the biocellulose may be commercially available cellulose.

The biocellulose microfibrils constituting the biocellulose microfibril network dispersion used in the present invention have a number-average diameter of 30 to 60 nm. When the microfibrils have a number-average diameter in the above range, it is advantageous for human application and advantageous for use as a cosmetic composition or quasi-drug composition. Particularly, when the diameter is 30 to 60 nm, it is possible to express a unique function without being smeared during application while forming a network structure.

In addition, the maximum diameter of the biocellulose microfibrils is 60 to 100 nm. When the microfibrils have the maximum diameter in the above range, it can be advantageously applied to the human body, for example, as a cosmetic composition or quasi-drug composition. If the maximum diameter exceeds 200 nm, there may be a problem of deteriorating the feeling of use, such as being smeared during application.

In the present invention, the length of biocellulose microfibrils may be similar to that of the biocellulose used as a raw material. That is, the microfibril used in the present invention maintains the length of the biocellulose used as a raw material, which means that there is substantially no difference from the length of the biocellulose used as the raw material. Substantially no difference means that the difference between the lengths is ±10% or less, ±5% or less, ±1% or less, ±0.1% or less, or ±0.01% or less. Accordingly, the manufacturing process of the biocellulose microfibril network dispersion used in the present invention may not include cutting the microfibrils.

The biocellulose microfibril network dispersion may be prepared through various methods known in the art. However, a top-down pre-treatment process for the micronization of biocellulose raw materials, such as cutting microfibrils, is not required.

The biocellulose microfibril network dispersion may be prepared in the following way:
i) dissolving an N-oxyl compound in water;
ii) adding and stirring biocellulose and an oxidizing agent to the resulting solution; and
iii) removing reaction products through a washing process.

In the above method, the N-oxyl compound is used as a catalyst for accelerating the oxidation reaction. As the N-oxyl compound, one or more of 2,2,6,6-tetramethyl-1-piperidine-oxy radical (TEMPO); 4-hydroxy TEMPO derivatives prepared by imparting suitable hydrophobicity by etherification of a hydroxyl group of 4-hydroxy-2,2,6,6-tetramethyl-1-piperidine-oxy radical (4-hydroxy TEMPO) with alcohol or esterification with carboxylic acid or sulfonic acid; or an azaadamantane-type nitroxyl radical may be selected, and most preferably, 2,2,6,6-tetramethyl-1-piperidine-oxy radical (TEMPO) may be used. The amount of the N-oxyl compound used is not limited as long as the amount of the catalyst can convert biocellulose into microfibrils. For example, about 0.01 to 100 mmol, preferably about 0.01 to 50 mmol, and more preferably about 0.05 to 30 mmol may be used for 1 g of cellulose-based dry raw material.

The oxidizing agent that may be used includes, for example, halogens, hypohalogenous acids, halogenous acids, perhalogenic acids or salts thereof, halogen oxides, or halogen peroxides, but it is preferable to use chlorine among halogens. The chlorine may be, for example, chlorine, hypochlorous acid, chlorous acid, perchloric acid or salts thereof, chlorine oxide or chlorine peroxide, and sodium hypochlorite. The sodium hypochlorite is particularly preferable because it is economical in terms of microfibril production costs and has a low environmental load. The amount of oxidizing agent used may be selected within a range capable of accelerating the oxidation reaction. For example, 0.5 to 500 mmol, preferably 0.5 to 50 mmol, and more preferably about 2.5 to 25 mmol may be used for 1 g of a cellulose-based dry raw material.

According to the manufacturing method, the manufacturing method of the biocellulose microfibril network dispersion may smoothly proceed the oxidation reaction even at room temperature of 15 to 30 °C. As the oxidation reaction proceeds, a carboxyl group is generated in the biocellulose structure to decrease the pH of the reaction solution. Therefore, for an efficient oxidation reaction, the reaction solution's pH must be maintained at 8 to 12, for example, about 10 to 11, by continuously adding an alkaline solution. The reaction time of the oxidation reaction may be appropriately set to, for example, 0.5 to 60 hours, 1 to 50 hours, and 24 to 48 hours, but the present invention is not limited thereto.

In the present invention, the biocellulose microfibril network dispersion maintains the structural and physicochemical characteristics and/or water dispersibility of microfibrils when added to the aqueous solution, and particularly, may be stably dispersed by retaining the natural network of biocellulose. Thus, in the present invention, the biocellulose microfibrils may be formulated as a water-dispersible composition. The 'water-dispersible composition' is a composition containing water as a solvent and refers to a composition in which water-dispersible biocellulose microfibrils are dispersed in an aqueous phase.

When the biocellulose microfibril network dispersion as described above is applied to a cosmetic composition, a uniform physical film is formed when applied to the skin. When a color cosmetic is used thereon, cleansing of the color cosmetic is facilitated due to the physical film's detachment power. Specifically, when a color cosmetic applied on the skin coated with a film of biocellulose microfibrils is cleansed, as the microfibrils contact water, they are entangled with each other in a network structure unit and detached together, thereby helping the cleansing of color cosmetics. Particularly, in biocellulose, a solid physical film may be formed even on the skin curves such as pores and wrinkles, and thus perfect cleansing is made possible without a strong detergent.

In the composition, the biocellulose microfibrils may be included in an amount of 0.001 to 10 parts by weight, for example, 0.01 to 5 parts by weight or 0.1 to 3 parts by weight, based on the total weight of the composition, and the microfibrils included in this case may be a dried form. When the composition contains less than 0.001 parts by weight of the biocellulose microfibrils, a dense microfibril film may not be formed when applied to the skin. When more than 10 parts by weight of the biocellulose microfibrils is contained, it is difficult to formulate due to the entanglement between microfibrils.

In the present invention, the cosmetic composition may further include a component having a (glass) transition temperature equal to or higher than a lukewarm water temperature in addition to the biocellulose microfibrils. The component having a (glass) transition temperature equal to or higher than a lukewarm water temperature may have an effect of helping the biocellulose microfibrils form a physical film. Usually, it is not particularly limited as long as it is a component that can be applied to a washable cosmetic composition to form a film.

The component having a (glass) transition temperature equal to or higher than a lukewarm water temperature is a component whose phase is transferred by temperature change. At room temperature (20 to 25°C), the component remains in a state of gel or no fluidity, but at the temperature of lukewarm water (30 to 40°C) or higher, the component changes to a state of sol or fluidity. The component may be a temperature-sensitive polymer selected from the group consisting of methylcellulose, hydroxyl propyl methyl cellulose, hydroxyl propyl cellulose, Pluronic, poly(N-isopropylacrylamide) (PNIPAAm), poloxamers, a polyethylene oxide (PEO)-polypropylene oxide (PPO)-polyethylene oxide (PEO) copolymer, a polyethylene glycol (PEG)-polylactic acid/glycolic acid copolymer (PLGA)-polyethylene glycol (PEG) copolymer, and mixtures thereof; or a methoxy polyethylene glycol-polycaprolactone (mPEG-PCL) block copolymer or a film-forming wax selected from the group consisting of polyethylene wax, ozokerite wax, candelilla wax, carnauba wax, ceresin wax, beeswax, and paraffin wax, but the present invention is not limited thereto.

In the present invention, the film-forming polymer and the component having a (glass) transition temperature equal to or higher than a lukewarm water temperature may be included in an amount of 0.0001 to 20 parts by weight, such as 0.001 to 10 parts by weight, 0.01 to 5 parts by weight, 0.05 to 3 parts by weight, and 0.1 to 1 part by weight based on the total weight of the composition. When the composition contains less than 0.01 parts by weight of the film-forming polymer or the component having a (glass) transition temperature equal to or higher than a lukewarm water temperature, a sufficiently uniform film may not be formed when applied to the skin. When more than 20 parts by weight of the film-forming polymer or the component having a (glass) transition temperature equal to or higher than a lukewarm water temperature is contained, it may be difficult to formulate due to agglomeration with film-forming polymer or biocellulose, or it may inhibit the formation of a solid physical film of biocellulose.

In the present invention, the lukewarm water temperature is a temperature that expands blood vessels and helps blood circulation to facilitate cleansing and exfoliation during skin cleansing, and maybe in the range of 25 to 45°C, for example, in the range of 30 to 40°C, but the present invention is not limited thereto.

In the following examples, the present inventors confirmed that, by including polyvinyl alcohol as a film-forming polymer in addition to biocellulose microfibrils in the base cosmetic composition, the film was formed more strongly, and desorption occurred more easily due to a reaction with water, resulting in a more excellent cleansing effect.

The cosmetic composition may have a formulation of cosmetic water such as softening cosmetic water or nourishing cosmetic water, or spray-type cosmetic water, emulsions such as facial lotions or body lotions, creams such as nourishing creams, moisture creams, or eye creams, essences, cosmetic ointments, sprays, gels, packs, sunscreens, makeup bases, liquid-type or spray-type foundations, powders, makeup removers such as cleansing lotions or cleansing oils; cleansers such as cleansing foam, soaps, or body washes etc., but the present invention is not limited thereto.

The cosmetic composition may further include a known moisturizing component in addition to the above-described active ingredients, and may also include additives commonly used in the art within a range that does not deteriorate the effect of the composition. The additives may include adjuvants and carriers commonly used in the field of cosmetology, such as pearling agents, thickeners, viscosity modifiers, pH adjusters, fatty substances, organic solvents, solubilizers, thickening and gelling agents, softeners, antioxidants, suspending agents, stabilizers, foaming agents, fragrances, surfactants, water, ionic or nonionic emulsifiers, fillers, sequestering and chelating agents, antiseptics, preservatives, vitamins, blockers, wetting agents, essential oils, dyes, pigments, hydrophilic or lipophilic active agents, lipid vesicles, or any other ingredients widely used in cosmetics.

When the formulation of the cosmetic composition is a paste, cream, or gel, animal oils, vegetable oils, wax, paraffin, starch, tragacanth, cellulose derivatives, polyethylene glycol, silicone, bentonite, silica, talc, or zinc oxide may be used as the carrier component.

In addition, when the formulation of the cosmetic composition is a solution or emulsion, a solubilizing agent or emulsifying agent such as water, ethanol, isopropanol, ethyl carbonate, ethyl acetate, benzyl alcohol, benzyl benzoate, propylene glycol, dipropylene glycol, butylene glycol, 1,3-butyl glycol oil, glycerol aliphatic ester, PEG-8, polyethylene glycol, or sorbitan may be used as the carrier component.

When the formulation of the cosmetic composition is a spray, a propellant such as chlorofluorohydrocarbon, propane, butane, or dimethyl ether may be additionally included.

The cosmetic composition may be used in a conventional manner, and the frequency of use may be varied according to the user's skin condition or taste.

When the biocellulose microfibrils in the cosmetic composition are applied, a uniform film is formed on the skin, and the film reacts with water to be desorbed, so that when the cosmetic composition is first applied before applying the color cosmetic, color cosmetics can be easily cleansed.

The effects of the cosmetic composition, biocellulose microfibrils, and ease of cleansing may be applied to the above-described method as they are.

Provided is a skin makeup method including the step of applying the makeup base cosmetic composition for skin to the skin before applying the color cosmetic.

Advantages and features of the present invention, and the method of achieving them will become apparent with reference to the experimental examples and manufacturing examples described below in detail.

### [Advantageous Effects]

A makeup base cosmetic composition for skin includes biocellulose microfibrils, and thus color cosmetics applied on the skin can be easily cleansed due to a dense network of a three-dimensional network structure formed by the microfibrils.

### [Description of Drawings]

FIG. 1 illustrates a result of confirming the effect of expressing makeup according to whether or not a cellulose dispersion is applied according to one embodiment.
FIG. 2 illustrates a result of confirming the effect of cleansing power according to whether or not a cellulose dispersion is applied according to one embodiment.
FIG. 3 illustrates a result of confirming cleansing power of makeup of a makeup base cosmetic composition according to one embodiment.
FIG. 4 illustrates a result of confirming cleansing power of makeup of a makeup base cosmetic composition including a film-forming polymer according to one embodiment.

### [Modes of the Invention]

Hereinafter, the present invention will be described in detail by the following examples. However, the following examples are merely illustrative of the present invention, and the present invention are not limited to the following examples.

### Manufacturing Example. Preparation of cellulose dispersion

A cellulose microfibril dispersion was prepared in the same manner as in a previous study, Korean Patent Publication No. 10-2017-0103698. The dispersion prepared as described above was prepared by dispersing in purified water. The dispersion was used in an amount of 1.5 parts by weight (dry) based on 100 parts by weight of purified water.

### Experimental Example 1. Effect of the expressive power of makeup according to application of cellulose dispersion

To test the expressive power of makeup according to whether or not cellulose dispersion is applied, the dispersion of Manufacturing Example was applied to the same area on the skin at a concentration of 2mg/2cm² and dried, and then a water-in-oil type foundation was applied at the same concentration (2mg/2cm²) on the same area on the skin.

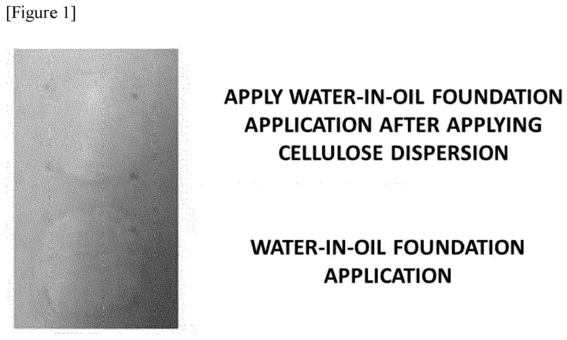

As a result, as shown in FIG. 1, even when the dispersion is applied and the foundation is applied thereon, It can be seen that color cosmetics are expressed without a sense of discomfort such as cracking, lifting, or clumping as when applied directly to the skin.

### Experimental Example 2. Confirmation of cleansing power according to application of cellulose dispersion

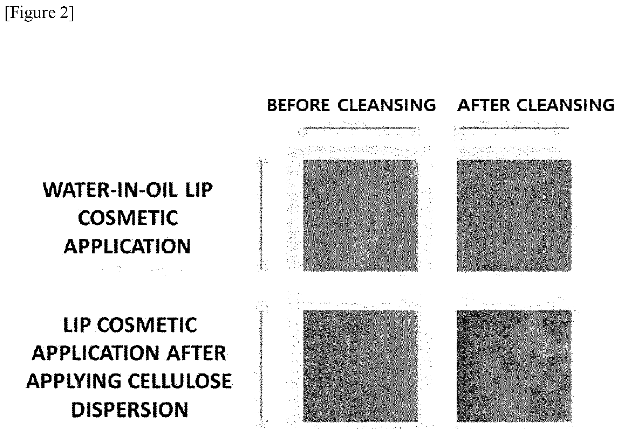

To confirm the cleansing power effect according to the application of the cellulose dispersion, the dispersion of Manufacturing Example was applied to the same area on the skin at a concentration of 2mg/2cm², dried, and then an oil-dispersed lip cosmetic was applied at the same concentration (2mg/2cm²) thereon. After applying the cosmetic, it was sufficiently dried and then cleansed using the same pressure with 30°C water. The cleansing results are shown in FIG. 2.

As a result, when the cellulose dispersion was applied, a cellulose microfibrous film was formed, and thus the cleansing power was remarkably increased.

### Examples 1 to 3. Preparation of oil-in-water emulsified base cosmetic composition

To confirm the cleansing power according to the concentration of the cellulose dispersion, a base cosmetic composition of an oil-in-water (O/W) emulsion formulation containing a cellulose dispersion was prepared according to the composition and content of Table 1 below. In this case, Examples 1 to 3 respectively refer to compositions including 10 parts by weight, 20 parts by weight, and 30 parts by weight of a raw material containing biocellulose microfibrils in an amount of 1.5 dry parts by weight based on 100 parts by weight of purified water.

**[Table 1]**

| **Ingredient name (content: parts by weight)** | **Example 1** | **Example 2** | **Example 3** | **Comparative Example 1** |
|---|---|---|---|---|
| Purified water | Up to 100 | Up to 100 | Up to 100 | Up to 100 |
| Trisodium EDTA | 0.02 | 0.02 | 0.02 | 0.02 |
| Carbomer | 0.2 | 0.2 | 0.2 | 0.2 |
| Acrylate/C10-C30 alkyl acrylate cross-polymer | 0.05 | 0.05 | 0.05 | 0.05 |
| Dipropylene glycol | 5 | 5 | 5 | 5 |
| 1,2-hexanediol | 1.5 | 1.5 | 1.5 | 1.5 |
| Biocellulose microfibril | 0.15 | 0.3 | 0.45 | - |
| Glycerin | 5 | 5 | 5 | 5 |
| Dimethicone | 4 | 4 | 4 | 4 |
| Cyclopentasiloxane | 1 | 1 | 1 | 1 |
| PEG-60 Hydrogenated Castor Oil | 0.4 | 0.4 | 0.4 | 0.4 |
| Tromethamine | 0.25 | 0.25 | 0.25 | 0.25 |
| Total | 100 | 100 | 100 | 100 |

### Experimental Example 3. Confirmation of cleansing power of base composition

To confirm the makeup cleansing power according to the application of the compositions of Examples 1 to 3, an experiment was performed in the following manner.

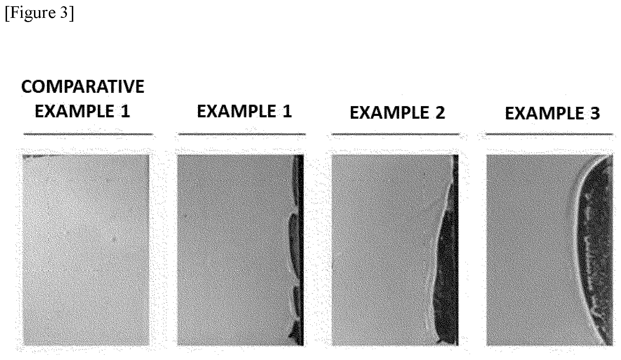

First, each of the compositions of Examples 1 to 3 was applied on a slide glass to a constant thickness. After drying for one day at room temperature for complete drying, a water-in-oil-type foundation was applied thereon to a constant thickness. After application, it was dried at room temperature for 4 hours and then immersed in 30 °C distilled water while stirring to check the cleansing effect. The composition of Comparative Example 1 was also tested in the same manner. The results of cleansing are shown in FIG. 3.

As shown in FIG. 3, when the composition of Comparative Example 1 containing no biocellulose microfibrils was applied, the foundation was hardly removed, whereas when the compositions of Examples 1 to 3 were used, the foundation was washed away. Particularly, when the biocellulose microfibrils' content in the formulation increased, a larger amount of the foundation was washed away. As a result, it was confirmed that when the content of the cellulose microfibrils increased, the cleansing power was also increased.

### Example 4. Preparation of oil-in-water emulsion base cosmetic composition containing film-forming polymer

To confirm the cleansing power according to biocellulose microfibrils in the presence of a film-forming agent which is generally known to aid cleansing, a base composition of an oil-in-water emulsion formulation containing a film-forming polymer and cellulose microfibrils was prepared according to the composition and contents shown in Table 2 below. In this case, polyvinyl alcohol was used as the film-forming polymer.

**[Table 2]**

| **Ingredients (content: parts by weight)** | **Comparative Example 2** | **Example 4** |
|---|---|---|
| Dipropylene glycol | 5.00 | 5.00 |
| Butylene Glycol | 3.00 | 3.00 |
| Triethylhexanoin | 1.50 | 1.50 |
| Dimethicone | 0.90 | 0.90 |
| Sucrose polytearate | 0.41 | 0.41 |
| Hydrogenated Polyisobutene | 0.09 | 0.09 |
| Polysorbate 60 | 0.70 | 0.70 |
| Purified water | Up to 100 | Up to 100 |
| Polyvinyl alcohol | 0.30 | 0.30 |
| Acrylate/C10-30 alkyl acrylate cross-polymer | 0.12 | 0.12 |
| Carbomer | 0.14 | 0.14 |
| Glycerin | 7.00 | 7.00 |
| 1,2-hexanediol | 1.30 | 1.30 |
| Trisodium EDTA | 0.03 | 0.03 |
| Biocellulose microfibril | 0.00 | 0.45 |
| Tromethamine | 0.22 | 0.22 |
| Silica | 0.70 | 0.70 |
| Total | 100 | 100 |

### Experimental Example 4. Confirmation of cleansing power of base composition containing film-forming polymer

To confirm whether the cleansing effect can be increased when the composition according to the present invention is used together with a film-forming polymer, which is well known to aid cleansing, an experiment was conducted in the following manner.

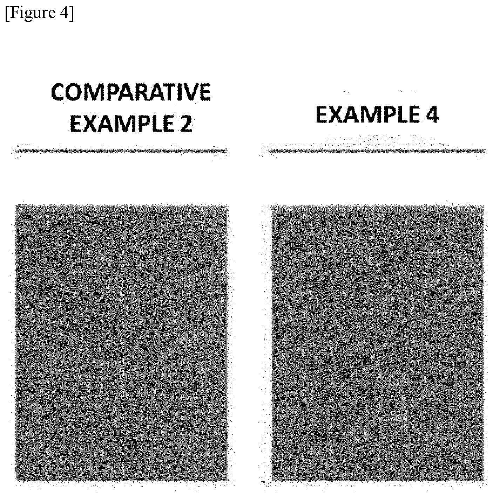

The composition of Example 2 was applied on a slide glass to a constant thickness. After drying for one day at room temperature for complete drying, a water-in-oil-type foundation was applied thereon to a constant thickness. After application, it was dried at room temperature for 4 hours and then immersed in 30°C distilled water while stirring to check the cleansing effect. The composition of Comparative Example 2 was also tested in the same manner. The results of cleansing are shown in FIG. 4.

From the results of Example 4 of FIG. 4, it was confirmed that the cellulose microfibrils can improve the ease of cleansing of the film-forming polymer which has been previously used for cleansing.

## Claims

1. A method of improving the removal efficiency of a color cosmetic, comprising applying a makeup base cosmetic composition for skin containing biocellulose microfibrils to the skin before applying the color cosmetic,
wherein the biocellulose microfibrils have a number-average diameter of 30 to 60 nm and a maximum diameter of 60 to 100 nm, and
wherein the cosmetic composition further comprises a polyvinyl alcohol-based film-forming polymer.

2. The method of claim 1, wherein a hydroxyl group of biocellulose is substituted with a carboxyl group, the biocellulose microfibrils are dispersed in an aqueous phase while forming a three-dimensional network structure.

3. The method of claim 1, wherein the biocellulose microfibrils are included in an amount of 0.001 to 10 parts by weight based on the total weight of the composition.

4. The method of claim 1, wherein the cosmetic composition further comprises:
a component having a glass transition temperature equal to or higher than a lukewarm water temperature or a component having a phase transition temperature equal to or higher than lukewarm water temperature,
wherein the lukewarm water temperature ranges from 25 to 45°C.

5. The method of claim 4, wherein:
the component having a glass transition temperature equal to or higher than a lukewarm water temperature is one or more temperature sensitive polymers selected from the group consisting of methylcellulose, hydroxyl propyl methyl cellulose, hydroxyl propyl cellulose, Pluronic, poly(N-isopropylacrylamide) (PNIPAAm), poloxamers, a polyethylene oxide (PEO)-polypropylene oxide (PPO)-polyethylene oxide (PEO) copolymer, a polyethylene glycol (PEG)-polylactic acid/glycolic acid copolymer (PLGA)-polyethylene glycol (PEG) copolymer, and a methoxypolyethylene glycol-polycaprolactone (mPEG-PCL) block copolymer; and
the component having the phase transition temperature equal to or higher than a lukewarm water temperature is one or more film-forming waxes selected from the group consisting of polyethylene wax, ozokerite wax, candelilla wax, carnauba wax, ceresin wax, beeswax, and paraffin wax.

6. The method of claim 1, wherein the cosmetic composition has an oil-in-water emulsion formulation.

7. The method of claim 1, wherein the cosmetic composition forms a physical film when applied to the skin.

8. The method of claim 1, wherein, in the method, a physical film formed by application of the cosmetic composition is desorbed, and thus the removal efficiency of the color cosmetic is improved.

## Patentansprüche

1. Verfahren zur Verbesserung der Entfernungseffizienz eines Farbkosmetikums, umfassend das Auftragen einer kosmetischen Zusammensetzung für Haut auf Makeup-Basis, die Biocellulose-Mikrofibrillen enthält, auf die Haut vor dem Auftragen des Farbkosmetikums,
wobei die Biocellulose-Mikrofibrillen einen zahlenmittleren Durchmesser von 30 bis 60 nm und einen maximalen Durchmesser von 60 bis 100 nm aufweisen, und
wobei die kosmetische Zusammensetzung ferner ein filmbildendes Polymer auf Polyvinylalkoholbasis umfasst.

2. Verfahren nach Anspruch 1, wobei eine Hydroxylgruppe von Biocellulose mit einer Carboxylgruppe substituiert ist, wobei die Biocellulose-Mikrofibrillen in einer wässrigen Phase dispergiert sind, während sie eine dreidimensionale Netzwerkstruktur bilden.

3. Verfahren nach Anspruch 1, wobei die Biocellulose-Mikrofibrillen in einer Menge von 0,001 bis 10 Gewichtsteilen, bezogen auf das Gesamtgewicht der Zusammensetzung, enthalten sind.

4. Verfahren nach Anspruch 1, wobei die kosmetische Zusammensetzung ferner Folgendes umfasst:
eine Komponente mit einer Glasübergangstemperatur, die gleich oder höher als eine Temperatur von lauwarmem Wasser ist, oder eine Komponente mit einer Phasenübergangstemperatur, die gleich oder höher als eine Temperatur von lauwarmem Wasser ist,
wobei die Temperatur von lauwarmem Wasser im Bereich von 25 bis 45 °C liegt.

5. Verfahren nach Anspruch 4, wobei:
die Komponente mit einer Glasübergangstemperatur, die gleich oder höher als eine Temperatur von lauwarmem Wasser ist, ein oder mehrere temperaturempfindliche Polymere ist, die aus der Gruppe ausgewählt sind, die aus Methylcellulose, Hydroxypropylmethylcellulose, Hydroxylpropylcellulose, Pluronic, Poly(N-isopropylacrylamid) (PNIPAAm), Poloxameren, einem Polyethylenoxid (PEO)-Polypropylenoxid (PPO)-Polyethylenoxid (PEO)-Copolymer, einem Polyethylenglycol (PEG)-Polymilchsäure/Glycolsäure-Copolymer (PLGA)-Polyethylenglycol (PEG)-Copolymer und einem Methoxypolyethylenglycol-Polycaprolacton (mPEG-PCL)-Blockcopolymer besteht; und
die Komponente mit der Phasenübergangstemperatur, die gleich oder höher als eine Temperatur von lauwarmem Wasser ist, ein oder mehrere filmbildende Wachse ist, die aus der Gruppe ausgewählt sind, die aus Polyethylenwachs, Ozokeritwachs, Candelillawachs, Carnaubawachs, Ceresinwachs, Bienenwachs und Paraffinwachs besteht.

6. Verfahren nach Anspruch 1, wobei die kosmetische Zusammensetzung eine Ölin-Wasser-Emulsionsformulierung aufweist.

7. Verfahren nach Anspruch 1, wobei die kosmetische Zusammensetzung einen physikalischen Film bildet, wenn sie auf die Haut aufgetragen wird.

8. Verfahren nach Anspruch 1, wobei in dem Verfahren ein physikalischer Film, der durch Auftragen der kosmetischen Zusammensetzung gebildet wird, desorbiert wird und somit die Entfernungseffizienz des Farbkosmetikums verbessert wird.

## Revendications

1. Procédé d'amélioration de l'efficacité d'élimination d'un cosmétique de couleur, consistant à appliquer une composition cosmétique de base de maquillage pour la peau contenant des microfibrilles de cellulose, sur la peau avant d'appliquer le cosmétique de couleur,
dans lequel les microfibrilles de cellulose ont un diamètre moyen en nombre de 30 à 60 nm et un diamètre maximum de 60 à 100 nm, et
dans lequel la composition cosmétique comprend en outre un polymère formant un film à base d'alcool polyvinylique.

2. Procédé selon la revendication 1, dans lequel un groupe hydroxyle de biocellulose est substitué par un groupe carboxyle, les microfibrilles de cellulose sont dispersées dans une phase aqueuse tout en formant une structure en réseau tridimensionnel.

3. Procédé selon la revendication 1, dans lequel les microfibrilles de cellulose sont inclues dans une quantité de 0,001 à 10 parties en poids sur la base du poids total de la composition.

4. Procédé selon la revendication 1, dans lequel la composition cosmétique comprend en outre:
un composant ayant une température de transition vitreuse égale ou supérieure à une température d'eau tiède ou un composant ayant une température de transition de phase égale ou supérieure à la température d'eau tiède,
dans lequel la température d'eau tiède est dans une plage de 25 à 45°C.

5. Procédé selon la revendication 4, dans lequel:
le composant ayant une température de transition vitreuse égale ou supérieure à une température d'eau tiède est un ou plusieurs polymères sensibles à la température sélectionnés dans le groupe constitué de méthylcellulose, cellulose d'hydroxypropyle méthyle, cellulose d'hydroxypropyle, Pluronic, poly(N-isopropylacrylamide) (PNIPAAm), poloxamères, un copolymère d'oxyde de polyéthylène (PEO)-oxyde de polypropylène (PPO)-oxyde de polyéthylène (PEO), un copolymère de polyéthylène glycol (PEG)-acide polylactique/acide glycolique (PLGA)-copolymère de polyéthylène glycol (PEG) et un copolymère séquencé de méthoxypolyéthylène glycol-polycaprolactone (mPEG-PCL); et
le composant ayant la température de transition de phase égale ou supérieure à une température d'eau tiède est une ou plusieurs cires formant un film sélectionnées dans le groupe constitué d'une cire de polyéthylène, une cire d'ozokérite, une cire de candelilla, une cire de carnauba, une cire de cérésine, une cire d'abeille et une cire de paraffine.

6. Procédé selon la revendication 1, dans lequel la composition cosmétique a une formulation émulsion huile-dans-eau.

7. Procédé selon la revendication 1, dans lequel la composition cosmétique forme un film physique quand elle est appliquée sur la peau.

8. Procédé selon la revendication 1, dans lequel, dans le procédé, un film physique formé par l'application de la composition cosmétique est désorbé et ainsi, l'efficacité d'élimination du cosmétique de couleur est améliorée.
